# EUROPEAN PATENT SPECIFICATION

(11) **EP 4 046 620 B1**
(45) Date of publication and mention of the grant of the patent: **08.04.2026**
(21) Application number: 21158687.0
(22) Date of filing: 23.02.2021
(51) Int. Cl.: A61K 8/06, A61K 8/60, A61K 8/92, A61Q 19/00

(54) **EMULSIONS COMPRISING MANNOSYLERYTHRITOL LIPID (MEL)**
EMULSIONEN ENTHALTEND MANNOSYLERYTHRITOL LIPID (MEL)
COMPOSITIONS COMPRENANT DU MANNOSYLERYTHRITOL LIPID (MEL)

(43) Date of publication of application: 24.08.2022
(73) Proprietor: Beiersdorf AG, 22529 Hamburg (DE)
(72) Inventor: Watanabe, Karin, Tokyo, 143-0023 O (JP); Alanya-Rousseau, Esma, 22527 Hamburg (DE); Perez Rivero, Christina, 20255 Hamburg (DE)
(74) Representative: Beiersdorf AG

(56) References cited:
- EP-A1- 2 662 068
- EP-A1- 3 510 992
- EP-A2- 2 749 651
- EP-B1- 2 578 204
- KR-A- 20160 060 975
- DATABASE GNPD [online] MINTEL; 19 December 2016 (2016-12-19), ANONYMOUS: "Nourishing Lip Care SPF 25 PA+++", XP055812369, retrieved from https://www.gnpd.com/sinatra/recordpage/4488083/ Database accession no. 4488083
- "Toning lotion", GNPD, MINTEL, 1 December 2016 (2016-12-01), XP002772933

## Description

The present invention relates to cosmetic skincare emulsions, in particular cosmetic facecare emulsions. The emulsions are stabilized by an emulsifier, which is obtained from renewable sources and produced by ecofriendly methods. The emulsions according to the invention show a good stability over a long time and quite a large temperature range.

The skin is the largest human organ and has many functions (for example, the organ of perception and temperature regulation). However, the most important function is the barrier function, which prevents the skin, and ultimately the entire organism, from drying out. At the same time, the skin protects from penetration and absorption of external substances, which may be toxic or otherwise harmful.

The skin is composed of layers, namely the epidermis, a basement membrane, the dermis and the subcutaneous tissue. The epidermis is the outmost layer consisting of Stratum Corneum, Stratum Lucidum, Stratum Granulosum, Stratum Spinosum and Stratum Basale. The corneocytes are the main cell type of the epidermis.

The Stratum Corneum (horny layer) is essential in establishing the barrier function of the skin. The "Elias" skin model, which is widely accepted in the field of dermatology and cosmetic (P. M. Elias, Structure and Function of the Stratum Corneum Permeability Barrier, Drug Dev. Res. 13, 1988, 97-105), describes the horny layer as a two-component system, similar to a brick wall (bricks and mortar model). In this model, the horny cells (corneocytes) correspond to the bricks, and the lipids in the intercellular spaces, which are of a complex composition, correspond to the mortar. This system is essentially a physical barrier to hydrophilic substances, but, because of its narrow and multilayered structure, also lipophilic substances can pass only to a limited extent.

Further structures contribute to the stability and function of the stratum corneum, hook-like structures of the corneocytes, corneo desmosomes, the bilayer of the stratum corneum and tight junctions.

Epidermal lipids contribute to the integrity of the horny layer. Epidermal lipids also affect the smoothness of the skin. In contrast to lipids secreted by the sebaceous glands, which do not form a continuous film on the skin, the epidermal lipids are evenly distributed over the entire horny layer.

The extremely complex interaction of moisture-binding substances and lipids in the upper layers of the skin is very important for the regulation of skin moisture. For this reason, cosmetics generally comprise, in addition to balanced lipid mixtures and water, water-binding substances.

The aim of cosmetic skin care is primarily to support the natural function of the skin as a barrier against environmental influences (e.g. dirt, chemicals, microorganisms) and against the loss of endogenous substances (e.g. water, natural fats, electrolytes). Cosmetic skin care shall strengthen and/or contribute to restore the natural condition of the skin.

If the barrier function is impaired, toxic or allergenic substances may by penetrate or microorganisms may infiltrate the skin, leading to toxic, allergic, or inflammatory reactions.

Another aim of skin care is to replenish lipids and water, which are lost by the daily washing routine. This becomes particularly important in cases, when the natural regeneration ability is impaired. Furthermore, skincare products should protect against environmental influences, in particular against sun and wind, and help to delay skin aging.

Frequently, skin care products are made available in form of an emulsion. Generally, emulsions are understood as a heterogeneous system of two liquids which are immiscible or miscible only to a limited extent with one another, which are usually referred to as phases. One liquid is present in form of droplets (disperse or internal phase), whilst the other one forms a continuous (coherent or internal) phase. The liquids (pure or as solutions) are present in an emulsion in a more or less fine distribution, which is generally of only limited stability.

If the two liquids are water and oil and oil droplets are finely dispersed in water, this composition is an oil-in-water emulsion (O/W emulsion, e.g. milk). The basic properties of an O/W emulsion are determined by the water phase, the basic properties being for example electrical conductivity, sensory properties, ability of the continuous phase to be stained.

In the case of a water-in-oil emulsion (W/O emulsion, e.g. butter), where droplets of water or an aqueous phase are finely dispersed in the oil phase, the basic properties are determined by the oil or oily phase.

In order to be able to ensure the stability of emulsions, interface-active substances, i.e. emulsifiers, are usually necessary. Emulsifiers may be of very different chemical nature. They may include silicone residues, or alkoxy residues, or may be of completely synthetic origin. All these residues may influence the biodegradability, which is becoming worse.

As the consumer becomes aware of the aspect of biodegradability, there is a need to provide cosmetic composition comprising as much raw materials as possible, which are biodegradable, at best all raw materials used being biodegradable.

The term biodegradability describes the process of the breakdown of organic compounds by living organisms, especially saprobionts. Ideally, inorganic substances such as CO₂, O₂ and ammonia are produced, compounds that are used by plants and microorganisms to build up organic compounds again.

Organic, chemical compounds that are readily biodegradable are classified according to OECD 301, for cosmetic compositions mostly OECD 301 B. These substances can be broken down quickly and completely.

Organic, chemical compounds that are classified according to OECD 302 are restricted, but basically biodegradable.

Another aspect becoming more and more important for consumers is the origin of the contained raw materials. It is preferred, if the raw materials are of natural origin or naturally derived.

The term "natural" in connection with natural ingredients for cosmetics is specified in ISO 16128; the first part of ISO 16128-1 was published on February 15, 2016, the second part of ISO 16128-2 was published in September 2017. According to this guideline, natural, cosmetic ingredients can be summarized as substances that are available from plants, animals, minerals or microorganisms, including those substances that are obtained from the above-mentioned sources by physical processes, fermentation processes (only those fermentation processes that are also used to occur in nature and lead to products that are also obtained in nature), and other manufacturing methods without chemical modification. The microorganisms may only be those that have not been genetically modified.

In general, nature-based substances are compounds that are derived from natural starting materials or are produced starting from natural starting materials. In order to assess the extent to which a substance is nature-based, the method for determining the "Biorenewable Carbon Index" (= BCI) may be used. This method is used to determine the percentage of carbon atoms that come from renewable plant or animal sources in relation to the carbon atoms from non-renewable, petrochemical sources, see HAPPI July, 58-60, 2009.

Hence, the problem of the present invention was to provide emulsions, especially O/W emulsions, which comprise an emulsifier and further raw materials, which are of natural origin or naturally derived. This may be described as to reduce the ecological footprint of said emulsions.

The problem could be solved by using mannosyl erythritol lipid (MEL) as an emulsifier. MEL may be described by the following structure: 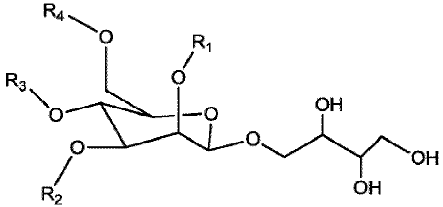
wherein R₁ and R₂ are identical or different, representing a saturated or unsaturated fatty acid; and
wherein R₃ and R₄ are identical or different, representing hydrogen atom or an acetic acid.

Depending on the presence of the hydrogen atom and/or the acetic acid residue, respectively, in the molecule four types of MEL may be differentiated:
R₃ and R₄, both are acetic acid, MEL-A,
R₄ represents acetic acid and R₃ represents hydrogen, MEL-B,
R₃ represents acetic acid and R₄ represents hydrogen, MEL-C,
R₃ and R₄, both are hydrogen.

MEL was described as a biosurfactant, which is obtained by fermentation processes. The document WO 2017/158193 A1 discloses a fermentation process using the yeasts *Pseudozyma antartica* or *Pseudozyma aphidis.* Advantageously, the carbon source is a vegetable oil, preferably soybean oil or rapeseed oil. Hence, MEL is a natural raw material.

The use of MEL in cosmetic composition is already disclosed in prior art.

EP 1964546 B1 discloses compositions containing MEL types A, B, and C as biosurfactant replacing ceramides. MEL is described for preventing skin roughness.

EP 3510992 A1 describes a skin whitening composition containing MEL as effective ingredient.

EP 3290500 A1 discloses compositions containing poly oxyalkylen carboxylates and a glycolipid, which may be MEL.

EP 3290020 A1 discloses compositions containing alkoxyalted fatty acid amides and a glycolipid, which may be MEL.

EP 2578204 B1 describes O/W emulsions containing emulsifiers free from alkoxy groups. The emulsifiers chosen for this purpose were highly hydrophilic and caused problems with regard to water resistance and stickiness. The addition of MEL as a co-surfactants could solve the problems.

The disclosures of prior could not point to the solution of the problem of the present invention, namely, to provide a storage- stable emulsion, preferably an O/W emulsion, with a pleasant skin feel and containing MEL as a single emulsifier only. In most of the emulsions disclosed in prior art, which contain MEL, a further emulsifier (co-emulsifier) is present or MEL is effective only as a co-emulsifier. Moreover, the emulsion of the present invention should contain at least 70 % natural or natural-derived raw materials, referring to all raw materials used, and at the same time should have the same properties as a similar emulsion containing classic raw materials, often derived from petrol sources or being (to a large extent) chemically modified.

Surprisingly, the problem was solved by a cosmetic emulsion, preferably an O/W emulsion containing
- the emulsifier mannosylerythritol lipid (MEL),
- at least one fluid emollient with a maximal interphacial tension to water at 20°C of 25 mN/m, and
- at least one wax having a melting temperature between 32 and 38°C, wherein in relation to the emulsifier(s) only MEL is contained.

According to the present invention MEL is contained as an emulsifier in the emulsion. In relation to the emulsifier MEL is the only emulsifier, which is contained in the emulsion according to the present invention. MEL is a natural substance obtained by a fermentation process using non-GMO (genetically modified organisms) organisms. Preferably, the emulsifier MEL is used as a raw material containing 95% or more of MEL, referring to the total weight of the raw material. Furthermore, it is preferred, if the raw material is a mixture of MEL-A, MEL-B, MEL-C, and MEL-D. Even furthermore, it is preferred, if the residues R₁ and R₂ (structure of MEL see above) are relatively short-chain fatty acids, namely fatty acids having 10 to 14 carbon atoms. A suitable raw material may be purchased from the company Oleon under the trade name Radiasurf ML 10, or Melavo from the company Labio, for example.

In the emulsion of the present invention the emulsifier MEL is contained in an amount of 0.1 to 2.0 % by weight, preferably 0.4 to 0.6 % by weight, referring to the total weight of the composition and referring to the active content.

In the emulsion of the present invention at least one fluid emollient with a maximum interphacial tension to water at 20°C of 25 mN/m is contained.

Emollients are known to act on the skin by softening and smoothing the skin. Frequently, emollients are chosen from lipophilic compounds, classic emollients are components such as lanolin, mineral oil and petroleum jelly, furthermore fats and waxes.

From the large list of components known to act as an emollient, only a selection of components has been shown to interact with the emulsifier MEL in a way that a stable emulsion is achieved. With regard to fluid emollients the selected components are characterized by a maximal interphacial tension to water at 20°C of 25 mN/m. However, it is preferred, if the interphacial tension to water is equal or less than 20 mN/m, more preferred equal or less than 15 mN/m.

The interphase tension to water was determined using a tensiometer (Tensiometer K100C, company Krüss GmbH). The interfacial tension of a liquid is measured against a second immiscible liquid. The resulting force is determined, which is required when molecules (located at the interface) interact on one hand with their own molecules and on the other hand with the molecules of a second liquid.

The interfacial tension to water of selected emollients is shown in the following table, the measurements were performed according to the method described above:

| **Emollient** | **Interfacial tension to water at 20°C, Measurement Start [mN/m]** | **Interfacial tension to water at 20°C, Measurement End [mN/m]** |
|---|---|---|
| Olive Oil, refined | 24,0 | 22,5 |
| Olive oil, fermented | <0,5 | <0,5 |
| Sunflower seed Hybrid Oil | 25,7 | 24,2 |
| Sunflower seed oil, fermented | <0,2 | <0,2 |
| AlgaPur (High Stability high oleic algae oil) | 14,5 | 14,5 |

The INCI name of AlgaPur (High Stability) is Triolein.

Refining refers to the process, by which impurities or other components that might have negative impact in the oil performance are removed from the oil.

In general, fermentation is the process, in which cells (microorganisms, plant or animal cells) are cultured in a bioreactor in liquid or solid medium to convert organic substances into biomass (growth) or into products, which may advantageously be used.

Referring to vegetable oils a fermentation process may follow the process of extracting the oil from the plants, especially the seeds or fruits. This fermentation process changes the qualities of the oil in most cases in a very advantageous manner. To illustrate this fact three oils, each in a refined and fermented condition were analyzed by gas chromatography. The respective oil samples were treated to generate the respective fatty acid methyl esters by known processes, the methyl esters were separated by gas chromatography and analyzed. The following table illustrates that the fermentation process does influence the composition of the respective oil, especially the content of monounsaturated (oleic acid) and polyunsaturated acids (linoleic acid).

| **Fatty acid** | **Sunflower Seed Oil High Oleic** | **Sunflower Seed Oil fermented** | **Olive Oil refined** | **Olive Oil fermented** | **High Stab. Oleic Algae Oil** | **Triolein** |
|---|---|---|---|---|---|---|
| C14:0 | 0.0 | 0.1 | 0.0 | 0.0 | 0.4 | 0.0 |
| C16:0 | 4.3 | 4.2 | 10.0 | 11.4 | 1.7 | 4.6 |
| C16:1 | 0.1 | 0.1 | 0.7 | 0.9 | 0.2 | 0.2 |
| C18:0 | 3.0 | 2.9 | 3.4 | 3.5 | 1.1 | 2.9 |
| **C18:1** | **82.7** | **80.7** | **77.2** | **74.8** | **93.0** | **81.0** |
| **C18:2** | **8.1** | **10.0** | **6.6** | **7.2** | **1.7** | **9.5** |
| C18:3 | 0.1 | 0.1 | 0.6 | 0.7 | 0.3 | 0.1 |
| C20:0 | 0.3 | 0.3 | 0.4 | 0.4 | 0.1 | 0.3 |
| C20:1 | 0.3 | 0.3 | 0.3 | 0.3 | 0.9 | 0.3 |
| C22:0 | 0.9 | 0.9 | 0.2 | 0.2 | 0.0 | 1.0 |
| C24:0 | 0.3 | 0.3 | 0.1 | 0.1 | 0.0 | 0.3 |
| Other | 0.0 | 0.2 | 0.5 | 0.7 | 0.6 | 0.0 |

Results of the gas chromatography analysis of the fatty acid methyl esters according to GTM GC006-11 with evaluation by means of the area percent (area %).

Sunflower seed oil is produced by pressing sunflower seeds. Fermented oils are vegetable oils, which have been subject of a fermentation process and thereby modified by the action of a microorganisms. This process may result in an increase in the amount of polyunsaturated fatty acids. Although polyunsaturated fatty acids are more easily prone to oxidation processes, especially linoleic acid is known as a beneficial agent for human skin. Therefore, an enrichment in this polyunsaturated fatty acid is desirable. For example, a suitable fermented sunflower seed oil may be purchased from the company of Labio under the trade name Fermentoil Sunflower seed.

Also, olive oil may be fermented, a suitable product may be purchased form the company Labio under the trade name Fermentoil Olive, for example.

AlgaPur High Stability high oleic algae oil is an algae oil. It is produced by microalgae grown on sugars. The microalgae accumulate intracellularly the oil during a fermentation process and the oil is later released by pressing the microalgae cells. Therefore, it is a fermented raw material having over 90% of omega-9 oleic acid with very low amounts of polyunsaturated fatty acids (<2%). The INCI name is triolein; to differentiate this algae oil from common triolein, the algae oil will be termed triolein obtained by a fermentation process.

According to the present invention it is preferred, if the at least one emollient is obtained by a fermentation process.

According to the present invention it is more preferred, if the at least one emollient is obtained by a fermentation process and has a maximum interphase tension to water at 20°C of 20 mN/m, advantageously a maximum interphase tension to water at 20°C of 15 mN/m.

It is most preferred, if olive oil, sunflower seed oil, and triolein, each obtained by a fermentation process according to the definition given in the context with of natural or nature-based substances, is contained in the emulsion of the present invention.

In the emulsion according to the present invention the at least one emollient with a maximal interphacial tension to water at 20°C of 25 mN/m is contained in a total amount of 5 to 15 % by weight, preferably 7 to 13 % by weight, referring to the total weight of the emulsion.

In the emulsion of the present invention at least one wax having a melting temperature between 32 and 38°C is contained.

"Waxes" or "wax-like substances" may be described substances that are plastic, solid to brittle, gross to fine crystalline, transparent to opaque, but not glassy at 20° C, melting without decomposition over 40° C. They have a comparatively low viscosity above their melting point, have a consistency and solubility that is highly dependent on temperature, and are polishable under gentle pressure (definition according to Roempp (online version; roempp.thieme.de/roempp4.0, last update referring to waxes being of December 2007)).

Waxes or wax-like substances are a chemically very different group of compounds. From this great variety of waxes, the at least one wax is chosen from only natural waxes of plant, animal and insect origin having a melting temperature between 32 and 38°C. Preferably the at least one wax is selected from the group of *Theobroma* Cacao seed butter, Shea butter, beeswax, and/or ceteraryl alcohol.

In the emulsion according to the present invention the at least one wax having a melting temperature between 32 and 38°C is contained in a total amount of 1 to 20 % by weight, preferably 7 to 15 % by weight, referring to the total weight of the emulsion.

Advantageously, according to the invention additionally at least one moisturizer is contained in the emulsion of the present invention. Moisturizers are hygroscopic substances, which bind water and thereby provide moisture. The hygroscopic quality is due to hydrophilic substituents of the molecule, in many cases hydroxyl groups, but other functional groups as amine or carboxyl groups have the same effect. Examples of humectants are propylene glycol, butylene glycol, glycerol, sorbitol, xylitol, maltitol, polydextrose, hydrogenated starch hydrolysate, urea, Aloe vera gel, alpha hydroxy acids such as lactic acid, honey. A preferred moisturizer is glycerol, which is obtained from natural processes.

If at least one moisturizer is contained in the emulsion of the present invention, the at least one moisturizer is preferably contained in a total amount of 1.0 to 10.0 % by weight, more preferably 2.0 to 8.0 % by weight, referring to the total weight of the emulsion.

Advantageously, according to the invention additionally at least one organic acid is contained in the emulsion of the present invention. It is preferred, if citric acid is contained. The addition of citric acid influences/adjusts the pH value of the emulsion of the present invention. It is preferred, if the pH value ranges between 4.0 and 6.5, preferably between 4.4 and 5.5.

If at least one organic acid is contained in the emulsion of the present invention, the at least one organic acid is preferably contained in a total amount of 0.1 to 1.0 % by weight, referring to the total weight of the emulsion.

Advantageously, according to the invention the emulsion shall be stable in relation to microbial contamination. In general, in order to reduce the number of microorganisms and prevent the growth of microorganisms preservatives are added to cosmetic compositions. However, many of the known preservatives are of synthetic origin, hence not natural.

In order to provide an emulsion, which is not spoiled by microorganisms and which does not contain any preservative of synthetic origin, the emulsion of the present invention is preserved by adding short chain alcohols, preferably chosen from ethanol and/or isopropanol, which are obtained from natural processes such as fermentation.

If at least one short chain alcohol is contained in the emulsion of the present invention, the at least one alcohol is preferably contained in a total amount of 3.0 to 7.0 % by weight, more preferably 4.0 to 6.0 % by weight, referring to the total weight of the emulsion.

Advantageously, according to the invention additionally at least one thickener is contained in the emulsion of the present invention. Thickeners are macromolecules, which have a mainly linear configuration and also have to a varying degree intermolecular forces of interaction permitting secondary and primary valence bonds between individual residues of the molecule and thus the formation of a reticular structure. Thickeners may be water-soluble natural or synthetic polymers, which form gels or viscous solutions in aqueous systems. They increase the viscosity of water by either binding water molecules (hydration) or else by absorbing and encapsulating the water into their macromolecules, which may be interwoven; both effects result in restricting the mobility of the water. Such water-soluble polymers represent a large group of chemically very different natural and synthetic polymers, whose common feature is their solubility in water or aqueous media. A precondition for the solubility in water is a sufficient high number of hydrophilic groups for ensuring the solubility in water and a limited crosslinking. The hydrophilic groups may be nonionic, anionic, or cationic groups.

More advantageously, the thickeners may be chosen from the following groups:
- organic, natural compounds, such as, for example, agar agar, carrageen, tragacanth, gum arabic, alginates, pectins, polyoses, guar flour, carob bean flour, starch, dextrins, gelatin, casein,
- organic, modified natural substances, such as, for example carboxymethylcellulose and other cellulose ethers, hydroxyethylcellulose and hydroxypropylcellulose and the like.

Even more advantageously the thickeners are biopolymers from various sources, most advantageously, the at least one natural or nature-based thickener may be selected from the group of xanthan gum, gellan gum, succinoglycan gum, scleroglucan, celluloses and/or derivatized celluloses.

For instance, Xanthan gum, an exopolysaccharide, is obtained Xanthomonas campestris. Xanthan gum is a polymer that consists of a main chain of β-1,4 glycosidically linked glucose molecules. Every second glucose residue has a side chain made up of two mannose residues, one glucoronic acid residue and pyruvate.

Xanthan gum dissolves well in hot and cold water, forming single and double helices. A three-dimensional network is created.

Xanthan gum can be obtained, for example, under the trade name Keltrol CG-RD from CP Kelco.

If at least one thickener is contained in the emulsion of the present invention, the at least one thickener is preferably contained in a total amount of 0.05 to 1.0 % by weight, more preferably 0.1 to 0.5 % by weight, referring to the total weight of the emulsion. The values are referring to the active content of the thickener.

The emulsion of the present invention is an aqueous composition. Preferably, water is contained in an amount of 60 to 90 % by weight, more preferably in an amount of 65 to 80 % by weight, referring to the total weight of the emulsion.

The emulsion of the present invention is preferably an O/W-emulsion. It is preferred, that the oil phase is contained in an amount of 10 to 30 % by weight, preferably in an amount of 15 to 27 % by weight. The amounts in % by weight are referring to the total weight of the emulsion.

The O/W-emulsion of the present invention may represent emulsions intended especially for day care or night care or intended for an all-day cream of the face.

If the emulsion is intended for day care, it may be preferred, if at least one UV-filter is contained. The at least one UV filter may be chosen from Titanium dioxide and/or Zinc oxide, which may be coated; triacine, butyl methoxydibenzolmethane and/or octocrylene.

It is also possible to incorporate a carotene, which may provide UV protection as well.

The composition of the present invention may be prepared by any technique known or effective to prepare an O/W emulsion or a W/O emulsion. The process to prepare the composition of the present invention comprises conventional formulating and mixing techniques.

To evaluate the storage stability the emulsions of the present invention were stored at different temperatures or at changing temperatures. The condition of the emulsion and the viscosity were checked. The criteria, which were analyzed, in order to evaluate the condition of the emulsion were discoloration, odor, oil separation, relative viscosity, stability, and water separation. For example the data of the composition according to example 1 are shown in the following table:

| | | | |
|---|---|---|---|
| Storage at -10°C | after 30 days | Discoloration | 100,00 |
| | | Odor | 100,00 |
| | | Oil separation | 100,00 |
| | | Rel. viscosity | 100,00 |
| | | Stability | 90,00 |
| | | Water separation | 100,00 |

| | | | |
|---|---|---|---|
| Storage at 40°C | after 14 days | Discoloration | 100,00 |
| | | Odor | 100,00 |
| | | Oil separation | 100,00 |
| | | Rel. viscosity | 100,00 |
| | | Stability | 100,00 |
| | | Water separation | 100,00 |
| | | | |
| | after 28 days | Discoloration | 100,00 |
| | | Odor | 100,00 |
| | | Oil separation | 100,00 |
| | | Rel. viscosity | 100,00 |
| | | Stability | 90,00 |
| | | Water separation | 100,00 |
| | | | |
| | after 60 days | Discoloration | 100,00 |
| | | Odor | 100,00 |
| | | Oil separation | 100,00 |
| | | Rel. viscosity | 100,00 |
| | | Stability | 80,00 |
| | | Water separation | 100,00 |
| | | | |
| | after 90 days | Discoloration | 100,00 |
| | | Odor | 100,00 |
| | | Oil separation | 100,00 |
| | | Rel. viscosity | 100,00 |
| | | Stability | 95,00 |
| | | Water separation | 100,00 |
| | | | |
| | after 120 days | Discoloration | 100,00 |
| | | Odor | 100,00 |
| | | Oil separation | 100,00 |
| | | Rel. viscosity | 100,00 |
| | | Stability | 100,00 |
| | | Water separation | 100,00 |
| | | | |
| | after 180 days | Discoloration | 100,00 |
| | | Odor | 100,00 |
| | | Oil separation | 100,00 |
| | | Rel. viscosity | 100,00 |
| | | Stability | 100,00 |
| | | Water separation | 100,00 |

| | | | |
|---|---|---|---|
| Storage at 50°C | after 14 days | Discoloration | 100,00 |
| | | Odor | 100,00 |
| | | Oil separation | 100,00 |
| | | Rel. viscosity | 100,00 |
| | | Stability | 80,00 |
| | | Water separation | 100,00 |
| | | | |
| | after 28 days | Discoloration | 100,00 |
| | | Odor | 100,00 |
| | | Oil separation | 100,00 |
| | | Rel. viscosity | 100,00 |
| | | Stability | 85,00 |
| | | Water separation | 100,00 |
| | | | |
| | after 60 days | Discoloration | 100,00 |
| | | Odor | 100,00 |
| | | Oil separation | 100,00 |
| | | Rel. viscosity | 100,00 |
| | | Stability | 75,00 |
| | | Water separation | 100,00 |
| Storage at 6°C | after 28 days | Discoloration | 100,00 |
| | | Odor | 100,00 |
| | | Oil separation | 100,00 |
| | | Rel. viscosity | 100,00 |
| | | Stability | 100,00 |
| | | Water separation | 100,00 |

| | | | |
|---|---|---|---|
| Storage at room temperature | after 1 day | Discoloration | 100,00 |
| | | Fragrance/ odor stability | 100,00 |
| | | Oil separation | 100,00 |
| | | Rel. viscosity | 100,00 |
| | | Stability | 100,00 |
| | | Water separation | 100,00 |
| | | | |
| | after 180 days | Discoloration | 100,00 |
| | | Fragrance/ odor stability | 100,00 |
| | | Oil separation | 100,00 |
| | | Rel. viscosity | 100,00 |
| | | Stability | 100,00 |
| | | Water separation | 100,00 |

### Analysis of pH value, composition according example 1:

| Storage at 25°C | pH value |
|---|---|
| after 1day | 5,17 |
| after 14 days | 5,18 |
| after 30 days | 5,23 |
| after 60 days | 5,08 |
| after 120 days | 5,03 |

### Analysis of the viscosity, composition according example 1:

| Storage and measurement at 25° | [mPa·s] |
|---|---|
| after 1 day | 2200,00 |
| after 14 days | 2950,00 |
| After 30 days | 2850,00 |
| After 60 days | 2500,00 |
| After 120 days | 2350,00 |

These data show, that the example composition 1, which is a composition according to the present invention, is highly stable with respect to stability of the color and the odor. There is no oil or water separation. The relative viscosity (rel. viscosity) describes the fluidity of the respective composition. The value 100 refers to a sample of composition 1, which is kept at room temperature.

Furthermore, the pH value keeps quite constant, only a variance of 2,7 %.

The viscosity values shown in the table above were determined with the viscosimeter proRheo Rheomat R 123 at 25 ° C, using spindle 1 or 2. The values show some variance, but the consistency of the composition remains very satisfying.

Unless otherwise stated, all tests and measurements were performed under "normal conditions". The term "normal conditions" refers to 20°C, 1013 hPa and a relative humidity of 50%.

### Examples:

The examples below are intended to illustrate the present invention without limiting it. The numerical values in the examples are percentages by weight, based on the total weight of the particular composition. The values refer to active content.

| **INCI name** | **1** | **2** | **3** | **4** |
|---|---|---|---|---|
| Mannosyerythritol Lipid | 0,475 | 0,25 | 1 | 0,8 |
| Aqua | 67,06 | 83,8953 | 58,5576 | 64,8215 |
| Glycerin | 6,97 | 1 | 7 | 5 |
| Citric Acid | 0,05 | 0,1 | 0,001 | 0,25 |
| Tocopherol | 0,01 | 0,0056 | 0,0111 | 0,0056 |
| Alcohol Denat. | 6,73 | 4 | 7 | 2 |
| Isopropyl Alcohol | 0,41 | 0,2469 | 0,4264 | 0,1218 |
| Xanthan Gum | 0,30 | 0,5 | 1 | 2 |
| Theobroma Cacao Seed Butter | 9,00 | 5 | 15 | 20 |
| t-Butyl Alcohol | 0,005 | 0,0022 | 0,0039 | 0,0011 |
| Triolein | 8,99 | 5 | 10 | 5 |

## Claims

1. Cosmetic emulsion, preferably an O/W emulsion containing
- the emulsifier mannosylerythritol lipid (MEL),
- at least one fluid emollient with a maximal interfacial tension to water at 20°C of 25 mN/m, measured by a tensiometer, and
- at least one wax having a melting temperature between 32 and 38°C
wherein in relation to the emulsifier(s) only MEL is contained.

2. Emulsion according to claim 1 and/or 2 **characterized in that** MEL is added as a raw material originated from a fermentation process.

3. Emulsion according to at least one of the preceding claims **characterized in that** MEL is added as a raw material containing at least 95 % MEL referring to the total weight of the raw material.

4. Emulsion according to at least one of the preceding claims **characterized in that** the raw material is a mixture of MEL-A, MEL-B, MEL-C, and MEL-D.

5. Emulsion according to at least one of the preceding claims **characterized in that** the residues R₁ and R₂ of the emulsifier MEL represent two fatty acid residues having 10 to 14 carbon atoms.

6. Emulsion according to at least one of the preceding claims **characterized in that** the emulsifier MEL is contained in an amount of 0.1 to 2.0 % by weight, preferably 0.4 to 0.6 % by weight, referring to the total weight of the composition and referring to the active content.

7. Emulsion according to at least one of the preceding claims **characterized in that** the at least one fluid emollient has interfacial tension to water is equal or less than 20 mN/m, preferably equal or less than 15 mN/m.

8. Emulsion according to at least one of the preceding claims **characterized in that** the at least one fluid emollient with a maximal interfacial tension to water at 20°C of 25 mN/m is chosen from fermented olive oil, fermented Sunflower seed oil, and/or triolein obtained by a fermentation process.

9. Emulsion according to at least one of the preceding claims **characterized in that** the at least one fluid emollient with a maximal interfacial tension to water at 20°C of 25 mN/m is contained in a total amount of 5 to 15 % by weight, preferably 7 to 13 % by weight, referring to the total weight of the emulsion.

10. Emulsion according to at least one of the preceding claims **characterized in that** the at least one wax having a melting temperature between 32 and 38°C is chosen from the group of *Theobroma* Cacao seed butter, Shea butter, beeswax, ceteraryl alcohol.

11. Emulsion according to at least one of the preceding claims **characterized in that** the at least one wax having a melting temperature between 32 and 38°C is contained in a total amount of 1 to 20 % by weight, preferably 7 to 15 % by weight, referring to the total weight of the emulsion.

12. Emulsion according to at least one of the preceding claims **characterized in that** additionally at least one moisturizer is contained, preferably chosen from propylene glycol, butylene glycol, glycerol, sorbitol, xylitol, maltitol, polydextrose, hydrogenated starch hydrolysate, urea, Aloe vera gel, alpha hydroxy acids such as lactic acid, and/or honey, more preferably glycerol, most preferably glycerol obtained by a fermentation process.

13. Emulsion according to at least one of the preceding claims **characterized in that** at least one short chain alcohol, preferably chosen from ethanol and/or isopropanol, more preferably ethanol and/or isopropanol which are obtained from a fermentation process.

14. Emulsion according to at least one of the preceding claims **characterized in that** additionally at least one thickener is contained, preferably chosen from a natural or nature-based thickener, more preferably from the group of xanthan gum, gellan gum, succinoglycan gum, scleroglucan, celluloses and/or derivatized celluloses, most preferably xanthan gum.

15. Emulsion according to at least one of the preceding claims **characterized in that** the emulsion is an O/W-emulsion.

## Patentansprüche

1. Kosmetische Emulsion, vorzugsweise Öl-in-Wasser-Emulsion, die Folgendes enthält
- den Emulgator Mannosylerythritol-Lipid (MEL),
- mindestens ein fluides Emolliens mit einer maximalen Grenzflächenspannung gegenüber Wasser bei 20 °C von 25 mN/m, gemessen mit einem Tensiometer, und
- mindestens ein Wachs mit einer Schmelztemperatur zwischen 32 und 38 °C, wobei im Hinblick auf den Emulgator bzw. die Emulgatoren nur MEL enthalten ist.

2. Emulsion nach Anspruch 1 und/oder 2, **dadurch gekennzeichnet, dass** MEL als Rohmaterial, das aus einem Fermentationsprozess stammt, hinzugefügt wird.

3. Emulsion nach mindestens einem der vorhergehenden Ansprüche, **dadurch gekennzeichnet, dass** MEL als Rohmaterial hinzugefügt wird, das mindestens 95 MEL, bezogen auf das Gesamtgewicht des Rohmaterials, enthält.

4. Emulsion nach mindestens einem der vorhergehenden Ansprüche, **dadurch gekennzeichnet, dass** das Rohmaterial ein Gemisch aus MEL-A, MEL-B, MEL-C und MEL-D ist.

5. Emulsion nach mindestens einem der vorhergehenden Ansprüche, **dadurch gekennzeichnet, dass** die Reste R₁ und R₂ des Emulgators MEL zwei Fettsäurereste mit 10 bis 14 Kohlenstoffatomen darstellen.

6. Emulsion nach mindestens einem der vorhergehenden Ansprüche, **dadurch gekennzeichnet, dass** der Emulgator MEL in einer Menge von 0,1 bis 2,0 Gew.-%, vorzugsweise 0,4 bis 0,6 Gew.-%, bezogen auf das Gesamtgewicht der Zusammensetzung und bezogen auf den Wirkstoffgehalt, enthalten ist.

7. Emulsion nach mindestens einem der vorhergehenden Ansprüche, **dadurch gekennzeichnet, dass** das mindestens eine fluide Emolliens eine Grenzflächenspannung gegenüber Wasser hat, die gleich oder kleiner als 20 mN/m, vorzugsweise gleich oder kleiner als 15 mN/m ist.

8. Emulsion nach mindestens einem der vorhergehenden Ansprüche, **dadurch gekennzeichnet, dass** das mindestens eine fluide Emolliens mit einer maximalen Grenzflächenspannung gegenüber Wasser bei 20 °C von 25 mN/m aus fermentiertem Olivenöl, fermentiertem Sonnenblumenkernöl und/oder durch einen Fermentationsprozess erhaltenem Triolein ausgewählt ist.

9. Emulsion nach mindestens einem der vorhergehenden Ansprüche, **dadurch gekennzeichnet, dass** das mindestens eine fluide Emolliens mit einer maximalen Grenzflächenspannung gegenüber Wasser bei 20 °C von 25 mN/m in einer Gesamtmenge von 5 bis 15 Gew.-%, vorzugsweise 7 bis 13 Gew.-%, bezogen auf das Gesamtgewicht der Emulsion, enthalten ist.

10. Emulsion nach mindestens einem der vorhergehenden Ansprüche, **dadurch gekennzeichnet, dass** das mindestens eine Wachs mit einer Schmelztemperatur zwischen 32 und 38 °C aus der Gruppe mit *Theobroma* cacao-Bohnen-Butter, Sheabutter, Bienenwachs, Cetearylalkohol ausgewählt ist.

11. Emulsion nach mindestens einem der vorhergehenden Ansprüche, **dadurch gekennzeichnet, dass** das mindestens eine Wachs mit einer Schmelztemperatur zwischen 32 und 38 °C in einer Gesamtmenge von 1 bis 20 Gew.-%, vorzugsweise 7 bis 15 Gew.-%, bezogen auf das Gesamtgewicht der Emulsion, enthalten ist.

12. Emulsion nach mindestens einem der vorhergehenden Ansprüche, **dadurch gekennzeichnet, dass** zusätzlich mindestens ein Feuchtigkeitsspender enthalten ist, vorzugsweise ausgewählt aus Propylenglykol, Butylenglykol, Glycerin, Sorbit, Xylit, Maltit, Polydextrose, hydriertem Stärkehydrolysat, Harnstoff, Aloe vera-Gel, alpha-Hydroxysäuren, wie Milchsäure, und/oder Honig, stärker bevorzugt Glycerin, am stärksten bevorzugt Glycerin, das durch einen Fermentationsprozess erhalten wird.

13. Emulsion nach mindestens einem der vorhergehenden Ansprüche, **dadurch gekennzeichnet, dass** mindestens ein kurzkettiger Alkohol, vorzugsweise ausgewählt aus Ethanol und/oder Isopropanol, stärker bevorzugt Ethanol und/oder Isopropanol, die aus einem Fermentationsprozess erhalten werden.

14. Emulsion nach mindestens einem der vorhergehenden Ansprüche, **dadurch gekennzeichnet, dass** zusätzlich mindestens ein Verdickungsmittel enthalten ist, vorzugsweise ausgewählt aus einem natürlichen oder naturbasierten Verdickungsmittel, stärker bevorzugt aus der Gruppe mit Xanthan-Gummi, Gellan-Gummi, Succinoglycan-Gummi, Scleroglucan, Cellulosen und/oder derivatisierten Cellulosen, am stärksten bevorzugt Xanthan-Gummi.

15. Emulsion nach mindestens einem der vorhergehenden Ansprüche, **dadurch gekennzeichnet, dass** die Emulsion eine O/W-Emulsion ist.

## Revendications

1. Émulsion cosmétique, de préférence émulsion H/E contenant
- l'émulsifiant mannosylerythritol lipid (MEL),
- au moins un émollient fluide ayant une tension interfaciale maximale à l'eau à 20 °C de 25 mN/m, mesurée par un tensiomètre, et
- au moins une cire ayant une température de fusion comprise entre 32 et 38 °C dans laquelle seule le MEL est contenu par rapport à l'émulsifiant ou aux émulsifiants.

2. Émulsion selon la revendication 1 et/ou 2, **caractérisée en ce que** le MEL est ajouté en tant que matière première provenant d'un processus de fermentation.

3. Émulsion selon au moins l'une des revendications précédentes, **caractérisée en ce que** le MEL est ajouté en tant que matière première contenant au moins 95 % de MEL par rapport au poids total de la matière première.

4. Émulsion selon au moins l'une des revendications précédentes, **caractérisée en ce que** la matière première est un mélange de MEL-A, MEL-B, MEL-C et MEL-D.

5. Émulsion selon au moins l'une des revendications précédentes, **caractérisée en ce que** les radicaux R₁ et R₂ de l'émulsifiant MEL représentent deux radicaux d'acides gras ayant 10 à 14 atomes de carbone.

6. Émulsion selon au moins l'une des revendications précédentes, **caractérisée en ce que** l'émulsifiant MEL est contenu en une quantité de 0,1 à 2,0 % en poids, de préférence de 0,4 à 0,6 % en poids, par rapport au poids total de la composition et par rapport au contenu actif.

7. Émulsion selon au moins l'une des revendications précédentes, **caractérisée en ce que** le au moins un émollient fluide présente une tension interfaciale à l'eau égale ou inférieure à 20 mN/m, de préférence égale ou inférieure à 15 mN/M.

8. Émulsion selon au moins l'une des revendications précédentes, **caractérisée en ce que** le au moins un émollient fluide ayant une tension interfaciale maximale à l'eau à 20 °C de 25 mN/m est choisi parmi l'huile d'olive fermentée, l'huile de tournesol fermentée et/ou la trioléine obtenue par un processus de fermentation.

9. Émulsion selon au moins l'une des revendications précédentes, **caractérisée en ce que** le au moins un émollient fluide ayant une tension interfaciale maximale à l'eau à 20 °C de 25 mN/m est contenu en une quantité totale de 5 à 15 % en poids, de préférence de 7 à 13 % en poids, par rapport au poids total de l'émulsion.

10. Émulsion selon au moins l'une des revendications précédentes, **caractérisée en ce que** la au moins une cire ayant une température de fusion comprise entre 32 et 38 °C est choisie dans le groupe du beurre de graines de cacao Theobroma, du beurre de karité, de la cire d'abeille, de l'alcool cétérarylique.

11. Émulsion selon au moins l'une des revendications précédentes, **caractérisée en ce que** la au moins une cire ayant une température de fusion comprise entre 32 et 38 °C est contenue en une quantité totale de 1 à 20 % en poids, de préférence de 7 à 15 % en poids, par rapport au poids total de l'émulsion.

12. Émulsion selon au moins l'une des revendications précédentes, **caractérisée en ce qu'**elle contient en outre au moins un agent hydratant, de préférence choisi parmi le propylène glycol, le butylène glycol, le glycérol, le sorbitol, le xylitol, le maltitol, un polydextrose, un hydrolysat d'amidon hydrogéné, l'urée, un gel d'Aloe vera, des alpha-hydroxyacides tels que l'acide lactique, et/ou le miel, plus préférablement le glycérol, le plus préférablement le glycérol obtenu par un processus de fermentation.

13. Émulsion selon au moins l'une des revendications précédentes, **caractérisée en ce qu'**au moins un alcool à chaîne courte, de préférence choisi parmi l'éthanol et/ou l'isopropanol, plus préférablement l'éthanol et/ou l'isopropanol qui sont obtenus par un procédé de fermentation.

14. Émulsion selon au moins l'une des revendications précédentes, **caractérisée en ce qu'**elle contient en outre au moins un épaississant, de préférence choisi parmi un épaississant naturel ou d'origine naturelle, plus préférablement dans le groupe de la gomme xanthane, la gomme gellane, la gomme de succinoglycane, le scléroglucane, les celluloses et/ou les celluloses dérivatisées, plus préférablement la gomme xanthane.

15. Émulsion selon au moins l'une des revendications précédentes, **caractérisée en ce que** l'émulsion est une émulsion H/E.
